# EUROPEAN PATENT APPLICATION

(11) **EP 1 529 528 A1**
(43) Date of publication of application: **11.05.2005**
(21) Application number: 03025690.3
(22) Date of filing: 07.11.2003
(51) Int. Cl.: A61K 31/4015, A61P 9/10, A61P 3/10, A61P 9/00

(54) **2-pyrrolidone derivatives and their use in protecting mammalian cells against oxidation stress**

(71) Applicant: Universitätsklinikum Freiburg, 79106 Freiburg (DE); Johannes- Gutenberg-Universität Mainz, 55099 Mainz (DE)
(72) Inventor: Feuerstein, Thomas, 79289 Horben (DE); Förstermann, Ulrich, 55270 Ober-Olm (DE); Li, Huige, 55122 Mainz (DE)
(74) Representative: Koepe, Gerd L., Dipl.-Chem.

(57) **Abstract**

The invention relates to the use of one or more than one of the compounds of the general formula (I) 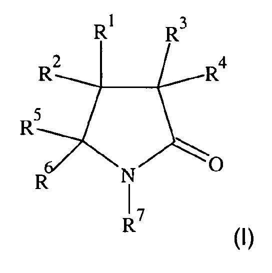 wherein R¹ and R² may be identical or different and, each independently of each other, represent hydrogen, hydroxy, amino, C₁ to C₁₀ alkyl, C₁ to C₁₀ alkoxy or C₁ to C₁₀ alkylamino, or R¹ and R², together with the carbon atom in position 4 of the pyrrolidinone ring, form a five- to ten-membered saturated or unsaturated ring which, besides the carbon atoms, may have up to 2 heteroatoms selected from oxygen, sulfur and nitrogen atoms, and which ring may be unsubstituted or substituted by up to 3 substituents selected from hydroxy groups, amino groups, C₁ to C₄ alkyl radicals, C₁ to C₄ alkoxy radicals and C₁ to C₄ alkylamino radicals, with the proviso that R¹ and R² are not both hydrogen; R³, R⁴, R⁵ and R⁶ may be identical or different and, each independently of each other, represent hydrogen, halogen, hydroxy, amino, C₁ to C₁₀ alkyl, C₁ to C₁₀ alkoxy, C₁ to C₁₀ alkylamino of C₆ to C₁₀ aryl; and R⁷ represents hydrogen, C₁ to C₁₀ alkyl or C₁ to C₁₀ acyl, and pharmaceutically acceptable salts thereof and/or prodrugs thereof for the manufacture of a medicament having an oxidation stress protection-promoting effect on mammalian cells.

## Description

The present invention relates to the use of 2-pyrrolidinone derivatives in the medical field, particular for the manufacture of medicaments promoting a protection of cells against oxidation stress.

### Background of the Invention

Some 1-, 3- and 5-substituted derivatives of 2-pyrrolidone are known as substances having anticonvulsant activity and/or possibly influencing glutamatergic transmission (J. Nakamura et al., Comparative Studies on the Anticonvulsant Activity of Lipophilic Derivatives of Gamma-Butyric Acid and 2-Pyrrolidinone in Mice; J. Pharmacobiodyn. 14 (1991), 1 - 8; P. A. Reddy et al., 3,3-Dialkyl- and 3-Alkyl-3-benzyl-substituted 2-Pyrrolidinones: A New Class of Anticonvulsant Agents, J. Med. Chem. 39 (1996), 1898 - 1906; M. P. De la Mora et al., Anticonvulsant Effect of 5-Ethyl-5-phenyl-2-pyrrolidinone and its Possible Relationship to γ-Aminobutyric acid-Dependent inhibitory mechanisms; Biochem. Pharmacol. 22 (1973), 2635 - 2639). The publication "Arzneimittelforschung 10 (1960), 243 - 250, discloses in Table I, No. XVII, the compound (II)

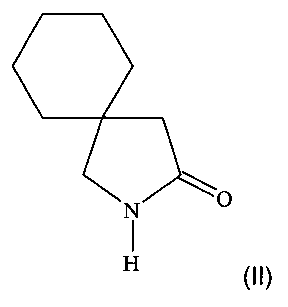

However, this compound is not regarded as particularly active, as is evident from the discussion in the right-hand column on page 249 of this publication, nor does the publication contain any reference to the use of this compound as a pharmaceutical.

Pharmaceuticals capable of influencing glutamatergic transmission and reducing the extracellular glutamate level and/or preventing the depolarization and overexcitation of postsynaptic cells, for example by presynaptically released glutamate, are disclosed in the document EP-A 1 032 560. The pharmaceuticals are based on 2-pyrrolidinone derivatives of the general formula (I) 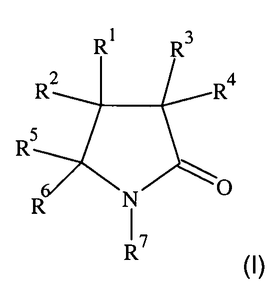 wherein R¹ and R², independently of each other, represent hydrogen, hydroxy, amino, C₁ to C₁₀ alkyl, C₁ to C₁₀ alkoxy or C₁ to C₁₀ alkylamino, or R¹ and R², together with the carbon atom in position 4 of the pyrrolidinone ring, form a five- to ten-membered saturated or unsaturated ring which, besides the carbon atoms, may have up to 2 heteroatoms selected from oxygen, sulfur and nitrogen atoms, and which ring may be unsubstituted or substituted by up to 3 substituents selected from hydroxy groups, amino groups, C₁ to C₄ alkyl radicals, C₁ to C₄ alkoxy radicals and C₁ to C₄ alkylamino radicals, with the proviso that R¹ and R² are not both hydrogen; R³, R⁴, R⁵ and R⁶, each independently of each other, represent hydrogen, halogen, hydroxy, amino, C₁ to C₁₀ alkyl, C₁ to C₁₀ alkoxy, C₁ to C₁₀ alkylamino of C₆ to C₁₀ aryl; and R⁷ represents hydrogen, C₁ to C₁₀ alkyl or C₁ to C₁₀ acyl.

The compounds of the above formula (I) comprise the compound of the above formula (II), if, for the compound of formula (I), the substituents R¹ and R² form a saturated unsubstituted 6-membered ring and all other substituents represent hydrogen. The latter compound (II) is named 8-aza-spiro[5,4]decan-9-one or Gabapentin-Lactam (GBP-L). All compounds of the general formula (I), including the compound of the above formula (II) (GBP-L), show an excellent effect in reducing the extracellular glutamate level and can therefore be used for the prophylaxis and treatment of disorders of the central nervous system such as stroke, hypoglycemia, hypoxia, trauma, epilepsy, Alzheimer's disease, AIDS-associated dementia, amyotrophic lateral sclerosis, Parkonson's disease and chronic alcoholism. The latter use of the above compounds was in detail described, inter alia, in the above document EP-A 1 032 560.

There is a growing awareness that oxidative stress plays a role in various pathophysiological conditions. The common risk factors for atherosclerosis increase production of reactive oxygen species (ROS) by endothelial, vascular smooth muscle, and adventitial cells. Elevated ROS levels have been implicated in the pathophysiology of cardiovascular diseases such as diabetes mellitus, atherosclerosis, and hypertension. Based on current knowledge, vascular ROS are generated by several important enzyme systems, including xanthine oxidase, nicotinamide adenine dinucleotide phosphate (NADPH) oxidases, and nitric oxide synthase.

Recently, also mitochondria have been implicated as an important source of ROS. For example, in cultured endothelial cells, hyperglycemia increases ROS production by mitochondria (T. Nishikawa et al., Normalizing mitochondrial superoxide production blocks three pathways of hyperglycaemic damage; Nature 404 (2000), 787 -790). Treatment with antioxidants ameliorates diabetic complications including the dysfunction of endothelial cells or increased platelet aggregation. Also glucocorticoid treatment has been shown to increase vascular ROS production, with mitochondria playing a major role (T. luchi et al., Glucocorticoid excess induces superoxide production in vascular endothelial cells and elicits vascular endothelial dysfunction; Circ. Res. 92, (2003), 81 - 87). Endothelial ROS production is also induced by cytokines and other factors released in atherosclerosis, such as TNF-α, interleukin-1β, angiotensin II, and interferon-γ (J. A. Vita et al., L-2-Oxothiazolidine-4-carboxylic acid reverses endothelial dysfunction in patients with coronary artery disease; J. Clin. Invest. 101 (1998), 1408 - 1414). In cardiomyocytes, openers of mitochondrial K_{ATP} channels have been shown to reduce damage caused by ROS (P. P. Dzeja et al., Targeting nucleotide-requiring enzymes: implications for diazoxide-induced cardioprotection; Am. J. Physiol. Heart Circ. Physiol. 284 (2003), H1048 - 1056). In neurons, the compound gabapentin-lactam has been shown to act as a specific opener of mitochondrial K_{ATP} channels (T. Jehle et al., Gabapentin-lactam (8-azaspiro[5,4]decan-9-on; GBP-L) inhibits oxygen-glucose deprivation-induced [3H]glutamate release and is a neuroprotective agent in a model of acute retinal ischemia; Naunyn Schmiedebergs Arch. Pharmacol. 362 (2000), 74 - 81).

Based on the above findings, it was investigated by the inventors whether Gabapentin-lactam can reduce ROS production in endothelial cells.

Coronary artery disease and its sequels - ischemia, myocardial infarction, and heart failure - are leading causes of morbidity and mortality in industrialized countries. Considerable effort has been devoted toward improving functional recovery and reducing the extent of infarction after ischemic episodes. As a step in this direction, it was found that the heart was significantly protected against ischemia-reperfusion (I/R) injury, if it was first exposed to brief ischemia or if a potassium channel opener was administered. These have been termed ischemic preconditioning and pharmacological preconditioning, respectively (K. D. Garlid et al., Mitochondrial potassium transport: The role of the mitochondrial ATP sensitive K(+) channel in cardiac function and cardioprotection; Biochim. Biophys. Acta 1606 ( 2003), 1 - 21).

It is well known that prolonged ("index") myocardial I/R leads to generation of ROS, the mitochondria being the major source of ROS (Q. Chen et al., Production of reactive oxygen species by mitochondria: central role of Complex III; J. Biol. Chem. 278 (2003), 36027 - 36031), and the resulting oxidation stress plays a central role in subsequent tissue damage and infarction. Preconditioning can extensively reduce infarct size and preserve myocardial function. Both the ischemic and pharmacological preconditioning were found to require an opening of a mitochondrial K_{ATP} channel (O. Oldenburg et al., P1075 opens mitochondrial K_{ATP} channels and generates reactive oxygen species resulting in cardioprotection of rabbit hearts; J. Mol. Cell Cardiol. 35 (2003), 1035 - 1042).

Previous investigations have discovered interesting mechanisms underlying cardiac preconditioning. Preconditioning (either by ischemia or by mitochondrial K_{ATP} openers) leads to a controlled generation of ROS by the mitochondria at threshold levels that do not damage the heart. These elevated levels of ROS are essential for activating signalling pathways leading to reduction of ROS burst that occurs during prolonged ("index") ischemia-reperfusion in a non-preconditioned heart and thus promote cardiac protection (Dzeja et al., loc. cit.; L. G. Kevin et al., Ischemic preconditioning alters real-time measure of O₂ radicals in intact hearts with ischemia and reperfusion; Am. J. Physiol. Heart Circ. Physiol. 284 (2003), H566-574). In other words, moderate increases in ROS during the preconditioning phase, which play an important second messenger role, mediate cardioprotection by reducing the excessive ROS generation during index I/R, which are associated with cell damage (Kevin et al., loc. cit.).

Such cardiac protection has been shown for mitochondrial K_{ATP} openers like diazoxide and P1075 (Dzeja et al., loc. cit.; Oldenburg et al. loc. cit.). Also bradykinin mimics ischemic preconditioning by generating ROS. Closing mitochondrial K_{ATP} channels with 5-hydroxydecanoate prevented increased ROS generation. Bradykinin preconditions through receptor-mediated production of nitric oxide which activates guanylyl cyclase. The resulting cGMP activates protein kinase G that opens mitoK_{ATP}. Subsequent release of ROS triggers cardioprotection (O. Oldenburg et al., Bradykinin induces mitochondrial ROS generation via NO, cGMP, PKG and mK_{ATP} channel opening and leads to cardioprotection; Am. J. Physiol. Heart Circ. Physiol. 2003, e-publication ahead of print).

Substances that prevent excessive ROS production under pathological conditions would be of great prophylactic and therapeutic interest. In addition, it would be desired that new applications of substances opening K_{ATP} channels be provided. Hence, it was an object of the present invention to provide substances that prevent excessive ROS production under pathological conditions. It was another object of the invention to provide substances which protect cells against oxidation stress, particularly against oxidation stress by excessive ROS production in the body. It was another object of the invention to provide substances, which protect cells against oxidation stress by exogenous sources of ROS.

It was also an object of the invention to provide substances the administration of which may contribute to reduce ROS production in endothelial cells. It was another object of the invention to provide the use of substances opening K_{ATP} channels. It was a further object of the invention to provide the use of K_{ATP} channel-opening substances for myocardial protection.

In accordance with the present invention, it was found that 2-pyrrolidinone derivatives of the above general formula (I) reduce oxidation stress in cells exerted by ROS production. In particular, it was found that gabapentin-lactam (GBP-L) of the general formula (II) above reduces ROS production in human vascular endothelium cells in a concentration-dependent manner.

A further surprising finding of the present invention was that 2-pyrrolidinone derivatives of the above general formula (I), and in particular GBP-L of the above general formula (II), achieve a myocardial protection by opening K_{ATP} channels.

Hence, the present invention relates to the use of one or more than one of the compounds of the general formula (I) 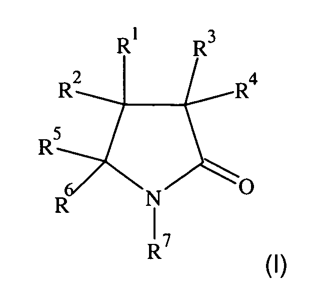 wherein R¹ and R² may be identical or different and, each independently of each other, represent hydrogen, hydroxy, amino, C₁ to C₁₀ alkyl, C₁ to C₁₀ alkoxy or C₁ to C₁₀ alkylamino, or R¹ and R², together with the carbon atom in position 4 of the pyrrolidinone ring, form a five- to ten-membered saturated or unsaturated ring which, besides the carbon atoms, may have up to 2 heteroatoms selected from oxygen, sulfur and nitrogen atoms, and which ring may be unsubstituted or substituted by up to 3 substituents selected from hydroxy groups, amino groups, C₁ to C₄ alkyl radicals, C₁ to C₄ alkoxy radicals and C₁ to C₄ alkylamino radicals, with the proviso that R¹ and R² are not both hydrogen;
R³, R⁴, R⁵ and R⁶ may be identical or different and, each independently of each other, represent hydrogen, halogen, hydroxy, amino, C₁ to C₁₀ alkyl, C₁ to C₁₀ alkoxy, C₁ to C₁₀ alkylamino of C₆ to C₁₀ aryl; and
R⁷ represents hydrogen, C₁ to C₁₀ alkyl or C₁ to C₁₀ acyl,
and pharmaceutically acceptable salts thereof and/or prodrugs thereof for the manufacture of a medicament having an oxidation stress protection-promoting effect on mammalian cells.

The invention also relates to a method of treating the mammalian body with the aim of promoting a protection of cells of said mammal against oxidation stress by administering a cell protection-promoting dose of a medicament comprising one or more than one of the compounds of the general formula (I) 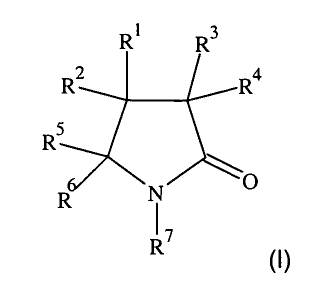 wherein R¹ and R² may be identical or different and, each independently of each other, represent hydrogen, hydroxy, amino, C₁ to C₁₀ alkyl, C₁ to C₁₀ alkoxy or C₁ to C₁₀ alkylamino, or R¹ and R², together with the carbon atom in position 4 of the pyrrolidinone ring, form a five- to ten-membered saturated or unsaturated ring which, besides the carbon atoms, may have up to 2 heteroatoms selected from oxygen, sulfur and nitrogen atoms, and which ring may be unsubstituted or substituted by up to 3 substituents selected from hydroxy groups, amino groups, C₁ to C₄ alkyl radicals, C₁ to C₄ alkoxy radicals and C₁ to C₄ alkylamino radicals, with the proviso that R¹ and R² are not both hydrogen;
R³, R⁴, R⁵ and R⁶ may be identical or different and, each independently of each other, represent hydrogen, halogen, hydroxy, amino, C₁ to C₁₀ alkyl, C₁ to C₁₀ alkoxy, C₁ to C₁₀ alkylamino of C₆ to C₁₀ aryl; and
R⁷ represents hydrogen, C₁ to C₁₀ alkyl or C₁ to C₁₀ acyl,
and pharmaceutically acceptable salts thereof and/or prodrugs thereof, to a mammal in need of such a treatment.

In a preferred embodiment, the invention relates to the use of the compound of the general formula (II) 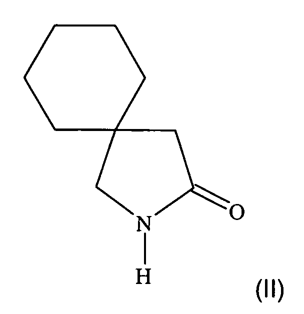 pharmaceutically acceptable salts thereof and/or prodrugs thereof, for the manufacture of a medicament having an oxidation stress protection-promoting effect on mammalian cells.

The invention, in another preferred embodiment, relates to a method of treating a mammalian body with the aim of promoting a protection of cells of said mammal against oxidation stress by administering a cell-survival-promoting dose of a medicament comprising a compound of the general formula (II) 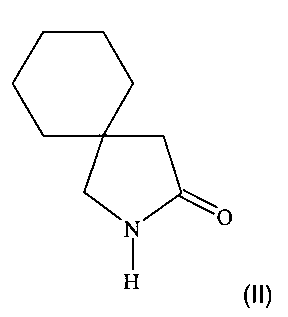 pharmaceutically acceptable salts thereof and/or prodrugs thereof to a mammal in need of such a treatment.

Further preferred embodiments of the use aspect of the present invention can be derived from the subclaims 2, 3 and 5 to 11.

Further preferred embodiments of the method of treatment aspect of the present invention can be derived from the subclaims 13, 14 and 16 to 22.

According to the present invention, the use of the compounds of the general formula (I), and even more preferred of GBP-L of the general formula (II), for the manufacture of a medicament having an effect, on mammalian cells, of protecting against oxidation stress exerted by reactive oxygen species (ROS) is preferred. Also preferred is, in another embodiment of the invention, a use of the compounds of the general formula (I), and even more preferred of GBP-L of the general formula (II), for the manufacture of a medicament having an effect, on mammalian cells, of opening mitochondrial K_{ATP} channels.

In preferred embodiments of the present invention, the use of the compound of the general formula (II), i. e. the use of GBP-L, as a compound having an oxidation stress protection-promoting effect on mammalian cells is particularly preferred.

The term "pharmaceutically acceptable salt", as used herein, relates to derivatives of the above compounds of the general formula (I) and of the formula (II) obtained by reacting the above compounds of the formulae (I) and (II) with acids to acid addition salts or with bases to basic addition salts. Only such addition salts are "pharmaceutically acceptable" which have the effect of promoting the oxidation stress protection, particularly the oxidation stress protection exerted by reactive oxygen species (ROS), and do not affect other functions of the mammalian cell in a negative way. Acid addition salts are more preferred according to the present invention. The acid addition salts can be prepared by reacting the compounds of the general formulae (I) and (II) with pharmaceutically acceptable acids. Illustrative and non-limiting examples of such acids are hydrochloric acid, acetic acid, succinic acid, tartaric acid, and others.

The term "prodrug", as used herein, relates to compounds which are precursors of the compounds of the general formula (I) and of the formula (II) in the pharmaceutical sense, i. e. may not (or may) be active ingredients having the same or a similar effect of compounds of the formulae (I) or (II) but are converted into an active (or more active) compound of the formulae (I) or (II) under natural conditions, e. g. in the mammalian body after administration to said mammal.

In accordance with the present invention, the use may comprise one compound of the general formula (I) or (II) or may comprise more than one of the compounds of the general formulae (I) or (II). Particularly preferred is the use of one single compound of the general formulae (I) or (II). Even more preferred is the use of GBP-L of the general formula (II).

The compounds used according to the present invention may be prepared in reaction sequences known from the prior art to the skilled person. With respect to suitable processes, reference may be made to the description of the above document EP-A 1 032 560 and the references addressed therein.

The compounds of the above formulae (I) and (II), in a preferred embodiment of the invention the compound of the formula (II), can be used, as was surprisingly found, for promoting a protection of cells against oxidation stress, in particular against oxidation stress exerted by ROS. As will be explained in detail in the examples, it was found that the compounds of the general formula (I), and in particular GBP-L of the general formula (II), act as openers of K_{ATP} channels which are located in the plasma membrane and also occur in the mitochondrial inner membranes of cells. The K_{ATP} channels play an important role in the mechanism of cardial and cerebral ischemic preconditioning. Drugs opening K_{ATP} channels mimick preconditioning so that the compounds of the present formula (I) and, peferably, GBP-L of the general formula (II), can be used in processes for the treatment of a mammalian body, especially of a human body, with the aim of promoting the protection, of said cells of said mammal or human, against oxidation stress. Such a process is characterized by administering a cell protection-promoting dose of a medicament comprising one or more than one compound of the general formula (I) or of the general formula (II) or its pharmaceutically acceptable salt(s) and/or prodrug(s) to said mammal or, even more preferred, to a human.

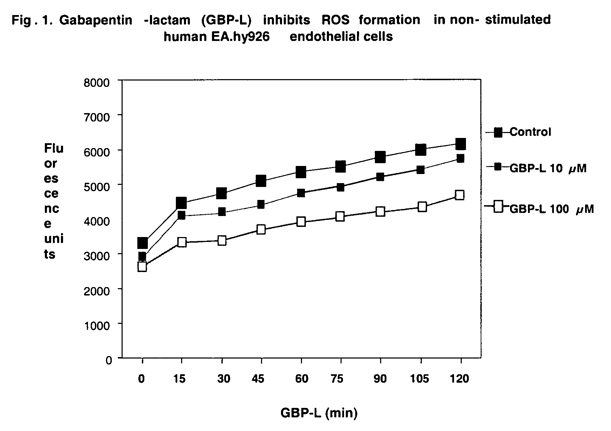

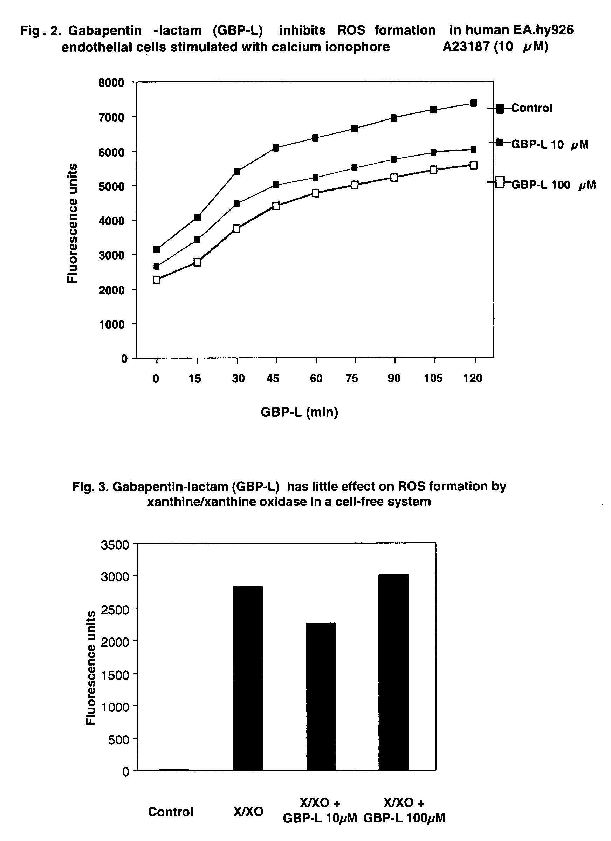

In another preferred embodiment of the invention, endothelial cells could surprisingly be protected against a damaging influence by free radicals by an administration of one or more than one compound of the general formula (I), and in particular by an administration of GBP-L of the general formula (II). In particular, the results obtained with human EA.hy926 endothelial cells indicate that gabapentin-lactam (GBP-L) indeed reduces ROS production in cultured human vascular endothelial cells in a concentration-dependent manner, as is shown in Figures 1 and 2.

Without wanting to be bound to a theory in accordance with the surprising findings of the invention, it is assumed that the ROS-inhibiting effect of gabapentin-lactam (GBP-L) results from a specific inhibition of ROS generation rather than from a non-specific radical scavenging property. In a cell-free system, gabapentin-lactam did not have any significant effect on ROS levels produced by xanthine/xanthine oxidase, as can be seen from Figure 3.

In a preferred embodiment of the invention, one or more than one compound of the general formula (I), and in particular GBP-L of the general formula (II), is/are used for the manufacture of a medicament, wherein the dosage of any of the compounds of the general formulae (I) and/or (II) is 0.01 to 5000 mg per day, administered in one or more than one dosage unit, preferably 0,1 to 4000 mg per day, even more preferred 1.0 to 1000 mg per day, administered in one or more than one dosage unit. It is preferred most to administer the above amount to a mammal, particularly to a human, in 3 dosage units per day.

Even further preferred is the use of one or more than one compound of the general formula (I), and in particular GBP-L of the general formula (II), for the manufacture of a medicament for the treatment of cardiovascular diseases, where it was much desired to achieve a protection of the cells against oxidation stress. Specific examples of medicaments which are manufactured by using the compounds of the general formula (I) in general and GBP-L of the general formula (II) specifically are medicaments for the prophylaxis or treatment of hypertension, diabetes mellitus, and/or artherosclerosis.

In another preferred use of one or more than one compound of the general formula (I), and in particular GBP-L of the general formula (II), medicaments for pharmacologically preconditioning myocardial cells are prepared. Such medicaments may induce pharmacological preconditioning of myocardial cells and may thereby protect the heart from e. g. reperfusion injury, in particular following a myocardial infarction, to mention just one exemplary, but not restricting case.

One or more than one compound of the general formula (I), and in particular GBP-L of the general formula (II), is/are used, in another preferred embodiment of the invention, for the manufacture of a medicament for administration by the oral, transdermal, intravenous, subcutaneous, intracutaneous, intramuscular and intrathecal routes, preferably by the oral or intravenous route.

When using the compounds of the above general formula (I), and particularly GBP-L of the above general formula (II), for the manufacture of medicaments, the resulting medicaments may contain, in addition to the above-mentioned compounds of the general formulae (I) and/or (II) or their pharmaceutically acceptable salts or their prodrugs, one or more than one suitable pharmaceutically acceptable vehicles, excipients, carriers, adjuvants, and/or auxiliary additives in order to bring them into a form suitable for the administration to a mammal, preferably to a human. Such pharmaceutically acceptable vehicles, excipients, carriers, adjuvants, and/or auxiliary additives are known per se to a person skilled in the pharmaceutical field and may be used dependent upon the requirements for achieving a suitable administration form and/or a dosage effective or a single case. They may be selected by a skilled person in accordance with few orienting tests, in accordance with the desired route of administration, in accordance with the disease to be treated (or from which to be protected) and with other usual parameters.

The invention is now exemplified, with reference to the figures, by the preferred embodiments of the following examples and comparative examples, which, however, are not restricting the invention to the specific embodiments.

In the Figures:
Figure 1 shows a graph resulting from a series of experiments wherein GBP-L of the general formula (II) was shown to inhibit ROS formation in non-stimulated human EA.hy926 cells in concentrations of GBP-L of 10 µM and 100 µM versus a control sample.
Figure 2 shows a graph resulting from a series of experiments wherein GBP-L of the general formula (II) was shown to inhibit ROS formation in human EA.hy926 cells, which were stimulated with calcium ionophore A23187 (10 µM) in concentrations of GBP-L of 10 µM and 100 µM versus a control sample.
Figure 3 shows a graph resulting from a series of experiments wherein GBP-L had little effect on ROS formation by xanthine/xanthine oxidase in a cell-free system.

In the following, the use of the compounds of the general formula (I) of the present invention is exemplified by describing the use of 8-aza-spiro[5,4]-decan-9-one (GBP-L) of the general formula (II). However, the present invention is not limited to the use of this compound or to a method with using this compound. GBP-L may be used instead of or together with other compounds of the general formula (I).

### Examples

To study whether GBP-L protects endothelial cells from oxidative stress, production of ROS was measured in human EA.hy 926 endothelial cells. The determination of intracellular ROS formation was based on the oxidation of 5-(and-6)-chloromethyl-2',7'-dichlorodihydrofluorescein diacetate (CM-H₂DCFDA) to yield an intracellular trapped fluorescent compound (H. Li et al., Dual effect of ceramide on human endothelial cells: Induction of oxidative stress and transcriptional upregulation of endothelial nitric oxide synthase; Circulation 106 (2002), 2250-2256). Cells loaded with 10 µM CM-H₂DCFDA were treated with or without GBP-L (10 and 100 µM), in absence (Figure 1) or in the presence (Figure 2) of the calcium ionophore A23187 (10 µM). The resulting fluorescence was measured in a FLUOStar^{R} Reader (BMG Labtechnologies) for up to 2 hours.

Both in unstimulated cells and in cells stimulated with A23187, GBP-L reduced ROS production in a concentration-dependent manner (Figures 1 and 2).

In order to find out whether this reduction results from an inhibition of ROS formation or from an unspecific ROS-scavenging action of GBP-L, an additional study was performed in a cell-free system. In 96-well plates, GBP-L (10 and 100 µM) was added to the ROS-producing system xanthine/xanthine oxidase (100 *µ*M and 1 mU/ml). ROS concentration was quantified by L-012 chemiluminescence (H. Y. Sohn et al., Sensitive superoxide detection in vascular cells by the new chemiluminescence dye L-012. J. Vasc. Res. 36 (1999), 456 - 464). As shown in Figure 3, GBP-L has no effect on pre-existing ROS, indicating that the ROS-inhibiting effect of gabapentin-lactam results from a specific inhibition of ROS generation rather than from non-specific radical scavenging properties.

These findings lead to the conclusion that GBP-L has a protective role in pathological conditions of the vasculature that involve ROS production, such as hypertension, diabetes mellitus, atherosclerosis and that GBP-L is an interesting agent for the prophylaxis or treatment of cardiovascular diseases associated with increased ROS production.

## Claims

1. Use of one or more than one of the compounds of the general formula (I) 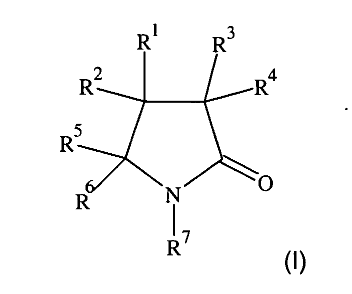 wherein R¹ and R² may be identical or different and, each independently of each other, represent hydrogen, hydroxy, amino, C₁ to C₁₀ alkyl, C₁ to C₁₀ alkoxy or C₁ to C₁₀ alkylamino, or R¹ and R², together with the carbon atom in position 4 of the pyrrolidinone ring, form a five- to ten-membered saturated or unsaturated ring which, besides the carbon atoms, may have up to 2 heteroatoms selected from oxygen, sulfur and nitrogen atoms, and which ring may be unsubstituted or substituted by up to 3 substituents selected from hydroxy groups, amino groups, C₁ to C₄ alkyl radicals, C₁ to C₄ alkoxy radicals and C₁ to C₄ alkylamino radicals, with the proviso that R¹ and R² are not both hydrogen;
R³, R⁴, R⁵ and R⁶ may be identical or different and, each independently of each other, represent hydrogen, halogen, hydroxy, amino, C₁ to C₁₀ alkyl, C₁ to C₁₀ alkoxy, C₁ to C₁₀ alkylamino of C₆ to C₁₀ aryl; and
R⁷ represents hydrogen, C₁ to C₁₀ alkyl or C₁ to C₁₀ acyl,
and pharmaceutically acceptable salts thereof and/or prodrugs thereof for the manufacture of a medicament having an oxidation stress protection-promoting effect on mammalian cells.

2. The use according to claim 1 for the manufacture of a medicament having an effect, on mammalian cells, of protecting against oxidation stress exerted by reactive oxygen species (ROS).

3. The use according to claim 1 or claim 2 for the manufacture of a medicament having an effect, on mammalian cells, of opening mitochondrial K_{ATP} channels.

4. The use according to any of the claims 1 to 3, wherein the compound of the general formula (I) is 8-aza-spiro[5,4]decan-9-one (GBP-L) of the general formula (II)

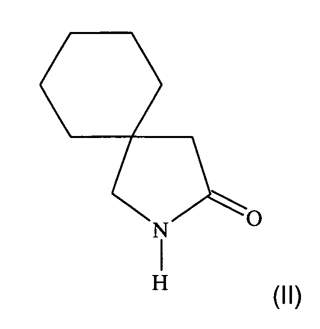

5. Use according to any of the claims 1 to 4, wherein the pharmaceutically acceptable salts of the compounds of the general formulae (I) and/or (II) are the hydrochlorides, acetates, succinates and/or tartrates of the compounds of the general formulae (I) and/or (II).

6. Use according to any of the claims 1 to 6, wherein the dosage of any of the compounds of the general formulae (I) and/or (II) is 0.01 to 5000 mg per day, administered in one or more than one dosage unit, preferably 0,1 to 4000 mg per day, even more preferred 1.0 to 1000 mg per day, administered in one or more than one dosage unit, even more preferred in 3 dosage units per day.

7. Use according to any of the claims 1 to 6 for the manufacture of a medicament for the prophylaxis or treatment of cardiovascular diseases.

8. Use according to any of the claims 1 to 7 for the manufacture of a medicament for the prophylaxis or treatment of hypertension, diabetes mellitus and/or artherosclerosis.

9. Use according to any of the claims 1 to 6 for the manufacture of a medicament for pharmacologically preconditioning myocardial cells.

10. Use according to any of the claims 1 to 6 and 9 for the manufacture of a medicament for protecting myocardial cells from reperfusion injury.

11. Use according to any of the claims 1 to 10 for the manufacture of a medicament for an administration by the oral, transdermal, intravenous, subcutaneous, intracutaneous, intramuscular, or intrathecal routes, preferably by the oral or intravenous route.

12. Method of treating the mammalian body with the aim of promoting a protection of cells of said mammal against oxidation stress by administering a cell protection-promoting dose of a medicament comprising one or more than one of the compounds of the general formula (I) 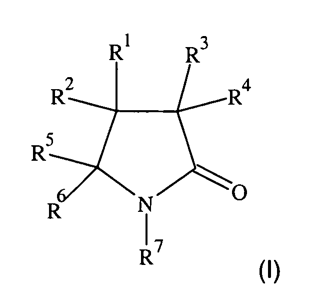 wherein R¹ and R² may be identical or different and, each independently of each other, represent hydrogen, hydroxy, amino, C₁ to C₁₀ alkyl, C₁ to C₁₀ alkoxy or C₁ to C₁₀ alkylamino, or R¹ and R², together with the carbon atom in position 4 of the pyrrolidinone ring, form a five- to ten-membered saturated or unsaturated ring which, besides the carbon atoms, may have up to 2 heteroatoms selected from oxygen, sulfur and nitrogen atoms, and which ring may be unsubstituted or substituted by up to 3 substituents selected from hydroxy groups, amino groups, C₁ to C₄ alkyl radicals, C₁ to C₄ alkoxy radicals and C₁ to C₄ alkylamino radicals, with the proviso that R¹ and R² are not both hydrogen;
R³, R⁴, R⁵ and R⁶ may be identical or different and, each independently of each other, represent hydrogen, halogen, hydroxy, amino, C₁ to C₁₀ alkyl, C₁ to C₁₀ alkoxy, C₁ to C₁₀ alkylamino of C₆ to C₁₀ aryl; and
R⁷ represents hydrogen, C₁ to C₁₀ alkyl or C₁ to C₁₀ acyl,
and pharmaceutically acceptable salts thereof and/or prodrugs thereof,
to a mammal in need of such a treatment.

13. The method according to claim 12, with the aim of promoting a protection of cells of said mammal against oxidation stress exerted by reactive oxygen species (ROS).

14. The method according to claim 12 or claim 13, with the aim of promoting an effect, on mammalian cells, of opening mitochondrial K_{ATP} channels.

15. The method according to any of the claims 12 to 14, wherein the compound of the general formula (I) is 8-aza-spiro[5,4]decan-9-one (GBP-L) of the general formula (II)

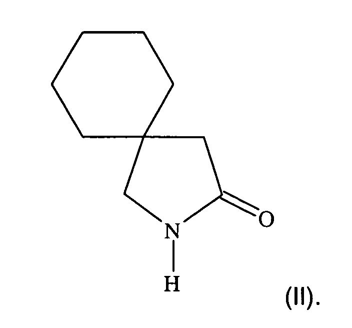

16. The method according to any of claims 12 to 15, wherein the hydrochlorides, acetates, succinates and/or tartrates of the compounds of the general formulae (I) and/or (I) are used as the pharmaceutically acceptable salts of the compounds of the general formulae (I) and/or (II).

17. The method according to any of claims 12 to 16, wherein one or more than one of the compounds of the formulae (I) and/or (II) are administered in an amount of from 0.01 to 5000 mg per day, administered in one or more than one dosage unit, preferably 0,1 to 4000 mg per day, even more preferred 1.0 to 1000 mg per day, administered in one or more than one dosage unit, even more preferred in 3 dosage units per day.

18. The method according to any of the claims 12 to 17 for the prophylaxis or treatment of cardiovascular diseases.

19. The method according to any of the claims 12 to 18 for the prophylaxis or treatment of hypertension, diabetes mellitus and/or artherosclerosis.

20. The method according to any of the claims 12 to 17 for pharmacologically preconditioning myocardial cells.

21. The method according to any of the claims 12 to 17 and 20 for protecting myocardial cells from reperfusion injury.

22. The method according to any of claims 12 to 21, wherein one or more than one of the compounds of the formulae (I) and/or (II) are administered by the oral, transdermal, intravenous, subcutaneous, intracutaneous, intramuscular, or intrathecal routes, preferably by the oral or intravenous route.
